# EUROPEAN PATENT SPECIFICATION

(11) **EP 2 659 923 B1**
(45) Date of publication and mention of the grant of the patent: **30.12.2020**
(21) Application number: 13166519.2
(22) Date of filing: 03.05.2013
(51) Int. Cl.: A61M 5/142, A61J 1/10, B65D 81/20, B65D 81/24

(54) **Pump and prefilled bag/anaesthesia - analgesia set**
Pumpe und vorgefüllter Beutel/Anästhesie - Analgesieset
Pompe et sac prérempli/ensemble d'anesthésie-analgésie

(30) Priority: 04.05.2012 GR 20120100242
(43) Date of publication of application: 06.11.2013
(73) Proprietor: Micrel Medical Devices S.A., Konstantinoupoleos 42 Karela Industrial Area 19441 Koropi (GR)
(72) Inventor: Tsoukalis, Achilleas, 15344 Gerakas (GR)
(74) Representative: Eisenführ Speiser

(56) References cited:
- EP-A1- 2 193 815
- FR-A1- 2 949 195
- US-A1- 2004 007 590
- US-A1- 2010 312 187

## Description

The present invention pertains to a system and a method for administering an anaesthetic and/or an analgesic drug to a patient comprising a pump for administering an anaesthetic and/or an analgesic drug preferably via the IV (intravenous) route or the central nerve block (spinal, epidural anaesthesia) or the peripheral nerve block and a storage bag containing the anaesthetic and/or the analgesic drug.

The FR2949195 relates to a bag for storing therapeutic solution comprising at least one compartment for receiving solution delimited by a membrane. The bag further comprises at least one appendage in the extension of the membrane and comprising an inscription zone. The therapeutic solution storage bag according to the invention makes it possible in particular to carry an inscription on the bag with a reduced risk of contamination.

The patent application US2010312187 describes a dispensing device for dispensing pain management medicaments to a patient comprising first and second threadably interconnectable sub-assemblies. The first of these sub-assemblies houses a novel collapsible fluid reservoir defining component while the second comprises a fluid delivery and control assembly that includes a novel flow control means that functions to control the flow of medicinal fluid from the fluid reservoir of the collapsible reservoir defining component toward the patient via a plurality of fluid flow control passageways.

The European paten application EP2193815A1 describes an infusion pump device comprising a customized bag/pump/set. Furthermore, it refers in to infusion devices for pain therapies. The system comprises a flexible container for storing a liquid medicament, with a wall consisting of two sheets of flexible material that are sealed together, comprises a storage volume, intended to contain the liquid medicament, and an access opening in fluid connection with the storage volume, intended to be fluidly connected to an infusion pump device.

Further reference is made to US2004/007590 which describes a drug/medical device system for administering a medical liquid to a patient. A bag is provided for containing a liquid intended to be supplied in a controlled manner to some other site, for example to a medical patient. The main bag portion has a pair of overlying side walls joined together around their edges, the side walls having integral extensions which together define an outlet region in communication with the interior of the main portion of the bag. The extensions are bonded together to define a tube-like passageway extending throughout the outlet region from the interior of the bag. The extensions are adapted to permit the connection thereto of a peristaltic pump device co-operable with the tube-like passageway to control the out-flow of liquid from the bag.

In the state of the art, a method for preparing and packaging a drug is provided as well as a method for the continuous and safe administration of same to patients. For example reference is made to US2012/0016295 or to its European counterpart EP 2 410 448 A2.

The most widespread pharmaceutically used substances in analgesia and/or in anaesthesia are generic, like morphine in all its forms (morphine sulphate, morphine hydrochloride), or oxycodone or ropivacaine, and henceforth are very low in price.

The packaging for long-term storage of such substances in analgesia and/or in anaesthesia for many years was glass, wherein glass offers protection against volatility and absorbency. During the last decade the production of multilayer sheets of plastic matter allowed the long-term plastics-packaging of drugs, i.e., for 2 or more years.

The drug storage bags of the infusion pump sets in the state of the art are made of a single layer flexible PVC sheet or a polypropylene sheet (named "Non-PVC") and may not be prefilled for long-term storage. Otherwise an alteration in drug concentration will occur, water-vapour exiting through the plastic and oxygen plus other oxide anion free radicals oxidants entering as time progresses, i.e. such drug storage bags exhibit poor long-term use and poor drug stability.

In the case of infusion with a portable pump, either a disposable infusion set is used bearing the pump manufacturer's spike plus some pharmaceutical company's drug prefilled bag which is the same for all pumps, portable or not, or during the treatment-application procedure to fill locally the bag supplied by the company supplying the pump, e.g. as in case of a Rythmic™ Full set of the Rythmic™ portable peristaltic pump offered by Micrel Medical Devices for direct or short-term use.

Prefilling for long-term storage of such an adapted to the pump bag, has size-associated advantages on account of the bag being manufactured in a shape and size adapted to the pump, this being an important advantage in portable use. It is common practice today for the commercial (made by the pharmaceutical companies) bags to be placed in banana- or shoulder-type pouches equipped with pump, in more than double the required size.

Another advantage is easy placement into the pump on account of the infusion set plus the drug comprising a single module, which is easily installed in place either in the pump cavity or as a cassette.

The infusion can be carried out in really portable conditions, in any pump-bag direction, if the pump bag is made of a material bearing micro-striations internally, in order to avoid the bag's walls collapsing and sticking together when the pump is in suction mode. When collapsing and sticking together of the walls occurs, bag emptying is prevented ending-up in under-infusion, which is not observed when the bag is vented and hanged vertically, e.g, by placing the bag on a pole, i.e., not in portable use. Prefilled bags are all supplied with a smooth internal surface since they are mainly used on poles connected to bedside pumps.

The localised filling of the bag (usually made of a single layer PVC film or polypropylene film) in the Hospital, at home or the small-quantity pharmaceutical compounding facility, bears the cost of syringes, gloves etc., plus the risks of nurse or formulator piercing. The stability duration of such filling in simple plastic sheets is only a few days, depending on the drug and the material, and a strategy for cost and risk reduction cannot be found. All of these risks should be avoided as will be reported analytically below in the context of the present invention.

In the practice of hospital treatment in-use errors are observed, wherein the wrong drug or drug concentration is infused, to the wrong patient, or the wrong protocol is followed, leading to patient apnoea and death. These risks should be avoided and the problem resolved by the present invention.

The high cost of analgesia for companies offering treatment at home, due to the fact that they have to carry the recently filled bag, which for compounding in PVC has a stability of only one to two weeks. Conversely, a high stability prefilled bag fitted with corresponding safety labelling/marking would allow bag collection from the local drugstore, with the respective nurse-cost saving.

It is the intention of the present invention to provide a pump system, fitted with a prefilled analgesia and/or anaesthesia bag, resolving all the above problems and affording the advantages in an easy and effective way.

### SUMMARY OF THE INVENTION

The invention is directed to an analgesia and/or to an anaesthesia drug/device system and an analgesic method and/or anaesthetic method utilizing a drug storage bag prefilled pharmaceutically or in a pharmaceutical compounding facility with a drug which system or method involves long-term stability of the drug prefilled storage bag, as defined in the claims, thus enabling the simplification of both, home treatment and hospital treatment procedures.

For example, the system or method is utilizing a storage bag comprising multi-layer films, drug-compatible, -with inner layers to constitute a barrier against any gases, e.g, oxygen, carbon dioxide, water vapour and/or humidity, and optionally ultraviolet radiation. This system and method comprises preferably a label/marking, providing information in a visual and/or wireless way to the pump system and the supporting services, directly or through the internet, in order to avoid medical errors regarding the administration of analgesic drugs. The system and method also enables for the unlocking of any drug prefilled storage bag, preferably a drug cassette, which is protected against abuse.

The invention preferably concerns an analgesics and/or an anaesthetics infusion pump combined with a drug-prefilled bag/set, wherein said term "prefilled" has the meaning as commonly used in a pharmaceutical authorization, e.g. the term is meant to define com pliance with regulatory requirements, preferably those set forth in the applicable guidelines of the European Medicine Agency (EMA, previously known as EMEA) and more preferably as additionally set forth with the applicable guidelines of the Food & Drug Administration (FDA) in the USA, for a new drug packaging (pharmaceutical envelope) having a longer shelf-life, particularly longer than one month, following a filling method in a pharmaceutical compounding facility designed for the exclusive cooperation between the storage bag and the pump which guarantees greater safety when infusing, in particular dangerous analgesics. Accordingly, the term "pre-filled" means that the bag or bag set is filled with the analgesic and/or the anaesthetic drug by a pharmaceutical company, in such way that it can be shelved and/or supplied by conventional pharmaceutical supply chain management. This means, in contrast to the state of the art, that the bag or bag set is not filled in a hospital, clinic or a pharmacy directly prior to individual administration to a patient, but can be produced in an industrial pharmaceutical manner more efficiently and on beforehand. Accordingly, using a multilayer bag, a compounding method of filling (e.g. by providing a compounder in each country) can offer the desired stability of analgesics and/or anaesthetics (long time on the shelf) so that storage at Hospital can be profitable avoiding burden and errors of local filling. Thus, the term "long-term" in the context of the invention is meant to designate a longer shelf-life of the drug-prefilled bag/set of at least 1 month, preferably at least 2 months, more preferably at least 3 months, and most preferably more than a year under standard room conditions.

In the present invention, this being novel over the state of the art, the disposable pump set (with or without a cassette) which may include the bag plus the infusion segment part (i.e., the piping alone) and/or the infusion mechanism, is considered after the pharmaceutical solution prefilling process as a drug/medical device combination and despite the fact that there is only drug distribution, it may be charged to the social security funds as a simple device without the drug, due to the low price of the analgesics and/or anaesthetics involved, or as the sum of both, drug plus device, or drug only in case it happens to be expensive.

In medical practice today such a drug/medical device combination is non existent, there exist bags/pump sets for filling locally, or drugs packed in small sacs linked to the pump set through a spike. Ease of use, error avoiding (i.e. wrong concentration) and lower cost, are the advantages offered by the present invention (set/drug on the shelf).

A part of the present invention is when the bag, in the final packaging, is not linked to the pump. It is preferred that in the full set assembly there is a sub-assembly (bag sub-assembly) comprising the bag and a simple connector e.g., luer connectors, fitting into the pump cavity/cassette, while the rest of the infusion set (second sub-assembly) can be reimbursed as usual. This way the sub-assembly parts may be charged (reimbursed) separately according to certain legislation. The label/marking contains information of a certain drug name with a certain date of expiry, concentration and solvent, for a certain pump family manufactured by a specific company. The present invention because of the long-term stability of the drug in the special bag reported below, and hence the long-term storage, provides the possibility and the advantage for the prefilling to be either carried out by the pharmaceutical company as a regular drug or in a pharmaceutical compounding facility, such that it has the right labelling/marking identifiable by the pump according to a special protocol as mentioned below, plus zero preparation errors as far as quantity and strict quality procedures are concerned, due to the organised production management system and not occasionaly on patient therapy demand .

### DETAILED DESCRIPTION OF THE INVENTION

The present invention pertains to a drug/medical device system for administering an analgesic drug and/or an anaesthetic drug to a patient preferably through the IV route or the central nerve block or the peripheral nerve block comprising:
(a) a pump for administering at least one analgesic drug and/or anaesthetic drug,
(b) a storage bag or a storage bag prefilled with the at least one analgesic drug and/or anaesthetic drug,
and characterized in that
the drug/device system comprising a drug storage bag pre-filled pharmaceutically or in a pharmaceutical compounding facility with a drug, and wherein the drug pre-filled storage bag has long-term stability with a shelf-life of the drug-prefilled bag/set of at least 1 month;
and wherein the drug storage bag is customized for a specific pump or pump group by at least one means whereby the pump (a) and the storage bag or storage bag set (b) are adjusted to exclusively cooperate with each other when administered to an individual patient, in that the system comprises a label/marking, providing information in a visual and/or wireless way to the pump system and the supporting services, directly or through the internet, in that the storage bag of (b) comprises a label/marking of one-dimensional or two-dimensional bar-code identification and/or RFID/NFC wireless identification of one or more data related to (i) drug type and drug concentration, (ii) solvent, (iii) bag volume, (iv) drug batch, (v) administration route, (vi) maximum limits for safe infusion per patient code, and optionally for (vii) wireless transmission of the infusion protocol; and wherein direct one to one information provision to the pump from the drug label/marking, concerning the (i) drug type and concentration enables the right and/or automatic choice of the infusion protocol;
and wherein the term "pre-filled" means that the bag or bag set is filled with the analgesic and/or the anaesthetic drug by a pharmaceutical company, and that it can be shelved and/or supplied by conventional pharmaceutical supply chain management, and wherein the pre-filled storage bag or a storage bag set (b) is provided in compliance with the guidelines of a competent drug regulatory authority applicable for pre-filled systems for long-term storage.

In the present invention, the drug bag customized for a specific pump or pump group is preferably made of multilayer plastic film, more preferably comprising (or consisting of) an outer layer made of EVA (Ethylene vinyl acetate) and/or polyolefins (preferably polypropylene, polyethylene) in contact with the drug , plus preferably further inner layers made of ethylene-Vinyl alcohol EVOH barrier, or AlOx (alumina oxide) - Al2O3, or SiOx, PCTFE (polychloro-triethylene) homopolymer, or CTFE (chlorotrifluoroethylene), polyacrylonitrile, or LCP liquid crystal polymers, or PVDC Polyvinylidene chloride, and/or a combination thereof, and/or ultraviolet barriers (UV filters) or radio-opaque packaging. Intermediate to the barrier layers there may be flexible plastic, adhesive -tie layers. Furthermore the surface finish of the layer in contact with the drug, preferably is not smooth; preferably it bears internal micro-striations or any other embossment or texture that makes the inner surface not completely flat in order to avoid the two bag sheets sticking together during suction, and for complete emptying in portable conditions. Next layers, numbers two to seven, preferably are made of EVOH and/or other materials acting as barriers against evaporation and the ingress of gases or UV radiation. This multilayer film material texture properties are similar to the simple monolayer film material since this multilayer film can be produced by co-extrusion process. A second production phase of the multilayer material in this invention can be the lamination to additional multilayer films.

Accordingly, it is preferred in the present invention that it pertains to a drug/medical device system, wherein the storage bag comprises internal striations, preferably internal micro-striations or any other type of ondulation/bumping that keeps the two layers at small distance to allow liquid to pass through under suction conditions as they do not collapse.

Accordingly, it is preferred in the present invention that it pertains to a drug/medical device system, wherein the storage bag is made of a multi-layer flexible sheetequipped with an additional transport barrier against oxygen, carbon dioxide, water vapour and/or humidity, and ultraviolet radiation.

Accordingly, it is preferred in the present invention that it pertains to a drug/medical device system, wherein the drug storage bag is packaged alternatively inside an aluminum/plastic laminated foil bag and Nitrogen gas Modified Atmosphere Packaging. As well known aluminum/plastic foil used in food packaging has best barrier characteristics with extremely low cost.

It is preferred according to the invention that the drug/medical device system comprises a protecting storage case made of cardboard or expanded polystyrene or other foam sheets so that the set has a mechanical strength protecting packaging.

Accordingly, it is preferred in the present invention that it pertains to a drug/medical device system, wherein the storage bag is adjusted for portable use, and more preferably wherein a single storage bag is equipped with an individual external packaging and/or a multitude of storage bags is equipped with a collective packaging,

Packaging of the set may be done in a hard-walled carton box, or with the protection provided by expanded polystyrene or other foam sheets plus an external simple label/marking according to the regulating environment of the drug-device combination.

Prefilling can be achieved by methods known to the person skilled in the art. For example the prefilling can be achieved in the following two ways:
In the normal pharmaceutical procedure, the bag's material is tested for stability (stability test) for every single drug, and its distribution in a prefilled form requires a complete marketing authorisation file, or a file for changes made in packaging, i.e., from material "a" to material "b", or the same material but in a different size, since drugs are generic supported by a different file. Pharmaceutical prefilling is preferably carried out under vacuum or modified atmosphere in an authorized pharmaceutical company under the supervision of the relevant authorities, observing all the necessary specifications and in a controlled--storage system for - controlled substances (in a locked warehouse).

In the second way, prefilling is meant as compounding, filling in a pharmaceutical compounding facility inside an already sterilized bag, preferably under vacuum or modified atmosphere conditions, wherein the test data (in specific authorized labs) show stability duration of more than one month, time considered to be necessary for the patient's benefits in distribution, the avoidance of unnecessary moves by the nursing staff and the destruction of expired prefilled drugs owing to the short stability duration. The present invention enables the normal operation of a pharmaceuticals warehouse, avoiding quick circulation (import-export) of drugs with short expiration times.

It is further preferred that the present invention pertains to a drug/medical device system, wherein the storage bag or the stoage bag set is in the form of a drug cassette. The combination of the safe cassette and the pump is described in more detail in a copending patent application.

The present invention also involves an analgesic and/or an anaesthetic drug. An analgesic drug, or as briefly also named an analgesic is any member of the group of drugs used to achieve analgesia, e.g. relief from pain. Commonly known as painkillers, analgesic drugs act in various ways on the peripheral and central nervous systems. They are distinct from anesthetics, which reversibly eliminate sensation, Analgesic drugs include paracetamol (known in the US as acetaminophen or simply APAP), the non-steroidal anti-inflammatory drugs (NSAIDs) such as the salicylates, and opioid drugs such as morphine and opium. In choosing analgesics, the severity and response to other medication determines the choice of agent. Analgesic choice is particularly also determined by the type of pain, which also may influence the route and/or mode of administration.

It is preferred in the present invention that it pertains to a drug/medical device system, wherein the analgesic drug is selected from the group of the analgesic pharmaceutical substances such as morphine, oxycodone, fentanyl, sufentanil, pethidine, and/or the anaesthetic drug is selected from the group of the amino-amide group of anaesthetic pharmaceutical substances such as ropivacaine, lidocaine, bupivacaine, and/or from the ester-based group of anaesthetic pharmaceutical substances such as cloroprocaine.

It is further preferred in the present invention that it pertains to a drug/medical device system, wherein the system comprises a patient's feedback function and interface by which the analgesic effect is reported by the patient as a value within a range, or as an answer to more complex questions asked by the feedback function.

It is further preferred in the present invention that it pertains to a drug/medical device system, wherein the system comprises a control function for the patient's identification and the administration route verification before administration start up, and for providing a commensurate control report.

It is further preferred in the present invention that it pertains to a drug/medical device system, wherein the system comprises a safety function for monitoring the breathing rhythm with an indication and/or warning in case of wrong dosage administration.

The present invention pertains to a drug/medical device system, wherein the storage bag comprises an one-dimensional or two-dimensional bar-code label/marking identification and/or RFID/NFC wireless identification of one or more data related to drug type, drug concentration, solvent, bag volume, drug batch, administration route, maximum limits for safe infusion per patient code, and optionally for wireless transmission of the infusion protocol. The term RFID has the meaning Radio Frequency Identifier, and the term NFC has the meaning Near Field Communication.

Radio-frequency identification (RFID) is the wireless non-contact use of radio-frequency electromagnetic fields to transfer data, for the purposes of automatically identifying and tracking tags attached to objects. Some tags require no battery and are powered and read at short ranges via magnetic fields (electromagnetic induction). Others use a local power source and emit radio waves (electromagnetic radiation at radio frequencies). The tag contains electronically stored information which may be read from up to several meters away. Unlike a bar code, the tag does not need to be within line of sight of the reader and may be embedded in the tracked object.

Near field communication (NFC) is a set of standards for electronic devices, and for example, is also used for smartphones and similar devices, to establish radio communication with each other by touching them together or bringing them into close proximity, usually no more than a few centimeters. Present and anticipated applications include contactless transactions, data exchange, and simplified setup of more complex communications such as Wi-Fi. Communication is also possible between an NFC device and an unpowered NFC chip, called a "tag". NFC standards cover communications protocols and data exchange formats, and are based on existing radio-frequency identification (RFID) standards including, for example, ISO/IEC 14443 and FeliCa. The NFC standards include, for example, ISO/IEC 18092 and those defined, promoted and certified for device compliance by the NFC Forum, which was founded in 2004, and now has more than 160 members.

It is further preferred in the present invention that it pertains to a drug/medical device system, wherein the system comprises a protection means against abuse and wherein the identification of the data content of the storage bag label/marking is carried out, preferably automatically, by a function of the pump, by a mobile application connected to or communicating with the pump locally, or by an internet application connected to or communicating with the pump locally, and wherein the integrated function of the pump, the mobile or internet application is enabled to unlock the storage bag, preferably the drug cassette, to be ready for use.

It is further preferred in the present invention that it pertains to a drug/medical device system, wherein system comprises a interface function through which the administration protocol, including but without limiting, the patient identification, the administration route, the drug label/marking content identification, the intermediate and/or final infusion protocol, the monitoring of analgesia level and/or other side-effect information, and the therapeutic results can be reported to a local network or the internet to the attending physician, and through which the attending physician may effect changes to the administration protocol within pre-set limits.

The RFID/NFC system found preferably on the bag label / marking allows the rapid stock report per commodity, with massive reading, and the use of a long-range antenna for storing, as well as rapid counting and invoicing of batches for sale.

Furthermore, the RFID/ NFC system mentioned below has preferably the ability to unlock a closed, hence safe, drug cassette only when it is placed on the pump and is authorized by the system for infusion. This way, safe drug cassettes may be stored at home, instead of the user procuring them, one by one, from the drugstore.

After prefilling and sterilization, a label or a marking is preferably placed on the backside with the same and/or additional information in order to avoid in-use errors, bearing a two-dimensional bar-code with hundreds of digital bytes stored in it, and/or chip with an RFID (Radio Frequency Identifier) or NFC (Near Field Communication) antenna operating without the use of a battery.

The information may include one or more of the following: drug type, concentration, type of solvent, bag volume, production lot in case an easy recall is required, highest flow and volume/safety limits for a specific patient code, preferred infusion protocol, administration route.

Reading labeldata may be executed through a RFID/NFC reader integrated into the infusion pump, or through a mobile application reading a bar-code via its photo camera feature, or NFC coming soon to all mobile phones enabling payments. A commercial mobile device, or one manufactured by our company, would be capable of transmitting label data via WiFi or GSM/GPRS to the internet and the treatment server, and via WiFi to the pump itself, the pump also including WiFi of particularly low consumption.

Direct one to one information provision to the pump from the drug label/marking, concerning the drug type and concentration enables the right and/or automatic choice of the infusion protocol, and the avoidance of errors being too frequent at present.

Furthermore, through the mobile application WiFi interface and the treatment server, additional information is arriving to the pump, relating to patient's name, his safety limits code, his identification through a direct question to the pump (are you patient Mr. XXX?), or patient bar-code, or identification of the patient's biometric data by the mobile application, and the final infusion protocol, and the control of the administration route by the patient. Patients are classified based on their age; weight etc., as regards their resistance to analgesics, and this code is used by the pump to compute safety infusion limits for each separate drug according to the information on the label/marking.

It is obvious that in utilizing the above automation that avoids (reduces) errors and may be carried out from the patient's setting, and the collection of the prefilled bag from the neighbourhood pharmacy store, and its easy aseptic placement (i.e., no special skills or license required) on the pump (comprising one set plus drug), by implementing the present invention, the high-cost transfer of nursing staff at home is no longer necessary, thus enabling money saving.

In addition, in the present invention a special mattress/sheet may be used having specific sensors for measuring heart beat, respirations per minute and spasms (pain). This layer is connected to the rest of the system, preferably via WiFi, and controls any tardiness or temporary loss of breath in order to sound an alarm or to lower the dose automatically, or through the intervention of a physician or both, i.e., automatically at first and then notifying the nursing staff for a definitive correction. In order to increase the dosage administered, the mattress feedback as well as questions addressed to the patient or his environment may be utilized.

## Claims

1. Drug/medical device system for administering an analgesic and/or an anesthetic drug to a patient preferably via the IV route or the central nerve block or the peripheral nerve block comprising
(a) a pump for administering at least one analgesic and/or anesthetic drug,
(b) a storage bag or a storage bag set pre-filled with the at least one analgesic and/or anaesthetic drug,
and **characterized in that**
the drug/device system comprising a drug storage bag pre-filled pharmaceutically or in a pharmaceutical compounding facility with a drug, and wherein the drug pre-filled storage bag has long-term stability with a shelf-life of the drug-prefilled bag/set of at least 1 month;
and wherein the drug storage bag is customized for a specific pump or pump group by at least one means whereby the pump (a) and the storage bag or storage bag set (b) are adjusted to exclusively cooperate with each other when administered to an individual patient, **in that** the system comprises a label/marking, providing information in a visual and/or wireless way to the pump system and the supporting services, directly or through the internet, **in that** the storage bag of (b) comprises a label/marking of one-dimensional or two-dimensional bar-code identification and/or RFID/NFC wireless identification of one or more data related to (i) drug type and drug concentration, (ii) solvent, (iii) bag volume, (iv) drug batch, (v) administration route, (vi) maximum limits for safe infusion per patient code, and optionally for (vii) wireless transmission of the infusion protocol; and wherein direct one to one information provision to the pump from the drug label/marking, concerning the (i) drug type and concentration enables the right and/or automatic choice of the infusion protocol;
and wherein the term "pre-filled" means that the bag or bag set is filled with the analgesic and/or the anaesthetic drug by a pharmaceutical company, and that it can be shelved and/or supplied by conventional pharmaceutical supply chain management, and wherein the pre-filled storage bag or a storage bag set (b) is provided in compliance with the guidelines of a competent drug regulatory authority applicable for pre-filled systems for long-term storage.

2. Drug/medical device system according to claim 1, wherein the storage bag is made of a multilayer film that has a surface in contact with the drug made of a material selected from the group of ethylenevinylacetate (EVA), and a linear polyolefinic polymer, and/or a cyclic polyolefinic polymer, preferably of a linear polyolefinic polymer; and further inner layers made of barrier materials to moisture, gases and UV light.

3. Drug/medical device system according to claim 1 or 2, comprising a protecting storage case made of cardboard or expanded polystyrene or other foam sheets.

4. Drug/medical device system according to any of the claims 1 to 3, wherein the storage bag comprises internal striations, preferably internal micro-striations, or any other embossment or texture that makes the surface in contact with the drug not completely flat.

5. Drug/medical device system according to any of the claims 1 to 4, wherein the storage bag is adjusted for portable use, and preferably wherein a single storage bag is equipped with an individual external packaging and/or a multitude of storage bags is equipped with a collective packaging,

6. Drug/medical device system according to any of the claims 1 to 5, wherein the storage bag or the storage bag set is in the form of a drug cassette.

7. Drug/medical device system according to any of the claims 1 to 6, wherein the analgesic drug is selected from the group of the analgesic pharmaceutical substances such as morphine, oxycodone, fentanyl, sufentanil, pethidine, and/or the anaesthetic drug is selected from the group of the amino-amide group of anaesthetic pharmaceutical substances such as ropivacaine, lidocaine, bupivacaine, and/or from the ester-based group of anaesthetic pharmaceutical substances such as cloroprocaine.

8. Drug/medical device system according to any of the claims 1 to 7, wherein the system comprises a patient's feedback function and interface by which the analgesic effect is reported by the patient as a value within a range, or as an answer to more complex questions asked by the feedback function.

9. Drug/medical device system according to any of the claims 1 to 8, wherein the system comprises a control function for the patient's identification and the administration route verification before administration start up, and for providing a commensurate control report.

10. Drug/medical device system according to any of the claims 1 to 9, wherein the system comprises a safety function for monitoring the breathing rhythm with an indication and/or warning in case of wrong dosage administration.

11. Drug/medical device system according to claim 1, wherein the system comprises a protection means against abuse and wherein the identification of the data content of the storage bag label/marking is carried out, preferably automatically, by a function of the pump, by a mobile application connected to or communicating with the pump locally, or by an internet application connected to or communicating with the pump locally, and wherein the integrated function of the pump, the mobile or internet application is enabled to unlock the storage bag, preferably the drug cassette, to be ready for use.

12. Drug/medical device system according to claim 11, wherein system comprises an interface function through which the administration protocol, including but without limiting, the patient identification, the administration route, the drug label content identification, the intermediate and/or final infusion protocol, the monitoring of analgesia level and/or other side-effect information, and the therapeutic results can be reported to a local network or the internet to the attending physician, and through which the attending physician may effect changes to the administration protocol within pre-set limits.

## Patentansprüche

1. Arzneimittel-/Medizinproduktsystem zur Verabreichung eines analgetischen (schmerzstillenden) und/oder eines anästhetischen (narkotisierenden) Arzneimittels an einen Patienten, bevorzugt über einen IV(intravenösen)-Zugang, oder einen zentralen Nervenblock, oder einen peripheren Nervenblock, umfassend
(a) eine Pumpe zur Verabreichung von mindestens einem analgetischen und/oder einem anästhetischen Arzneimittel,
(b) einen Aufbewahrungsbeutel oder ein Aufbewahrungsbeutelset, der/das mit dem mindestens einen analgetischen und/oder anästhetischen Arzneimittel vorgefüllt ist,
und **dadurch gekennzeichnet, dass**
das Arzneimittel-/Vorrichtungssystem einen pharmazeutisch oder in einer pharmazeutischen Einrichtung zur patientenindividuellen Herstellung von anwendungsfertigen Infusionslösungen (Compounding-Einrichtung) mit einem Arzneimittel vorgefüllten Arzneimittelaufbewahrungsbeutel umfasst, und wobei der mit einem Arzneimittel vorgefüllte Aufbewahrungsbeutel eine Langzeitstabilität mit einer Haltbarkeit des mit einem Arzneimittel vorgefüllten Beutels/Sets von mindestens 1 Monat aufweist;
und wobei der Arzneimittelaufbewahrungsbeutel durch mindestens ein Mittel individuell für eine spezielle Pumpe oder Pumpengruppe angepasst ist, wodurch die Pumpe (a) und der Aufbewahrungsbeutel oder das Aufbewahrungsbeutelset (b) angepasst sind, um beim Verabreichen an einen individuellen Patienten ausschließlich miteinander zusammenzuwirken, indem das System ein/e Etikett/Kennzeichnung umfasst, das/die Informationen in einer optischen und/oder drahtlosen Weise, direkt oder über das Internet, an das Pumpensystem und die unterstützenden Dienstleistungen bereitstellt, indem der Aufbewahrungsbeutel von (b) ein/e Etikett/Kennzeichnung einer eindimensionalen oder zweidimensionalen Strichcodekennzeichnung und/oder drahtlosen RFID/NFC(Radio Frequency Identification/Near Field Communication - Funkfrequenzerkennung/Nahfeldkommunikation)-Erkennung von einem oder mehreren Daten im Zusammenhang mit (i) einem Arzneimitteltyp und einer Arzneimittelkonzentration, (ii) einem Lösemittel, (iii) einem Beutelvolumen, (iv) einer Arzneimittelcharge, (v) einem Verabreichungsweg, (vi) Höchstgrenzen für die sichere Infusion pro Patientencode, und wahlweise für (vii) eine drahtlose Übermittlung des Infusionsprotokolls umfasst; und wobei eine direkte Eins-zu-Eins-Bereitstellung von Information von dem/der Arzneimitteletikett/-kennzeichnung an die Pumpe, die (i) die Arzneimittelart und die Konzentration betrifft, die richtige und/oder automatische Auswahl des Infusionsprotokolls ermöglicht;
und wobei der Begriff "vorgefüllt" bedeutet, dass der Beutel oder das Beutelset durch ein pharmazeutisches Unternehmen mit dem analgetischen und/oder dem anästhetischen Arzneimittel befüllt ist, und dass er/es gelagert und/oder durch herkömmliches pharmazeutisches Lieferkettenmanagement geliefert werden kann, und wobei der vorgefüllte Aufbewahrungsbeutel oder ein Aufbewahrungsbeutelset (b) in Übereinstimmung mit den geltenden Richtlinien einer zuständigen Arzneimittelaufsichtsbehörde für vorgefüllte Systeme für die Langzeitaufbewahrung bereitgestellt wird.

2. Arzneimittel-/Medizinproduktsystem nach Anspruch 1, wobei der Aufbewahrungsbeutel aus einer mehrlagigen Folie hergestellt ist, die eine Oberfläche in Kontakt mit dem Arzneimittel aufweist, die aus einem Material hergestellt ist, das ausgewählt ist aus der Gruppe von Ethylenvinylacetat (EVA), und einem linearen Polyolefin-Elastomer, und/oder einem zyklischen Polyolefin-Polymer, vorzugsweise aus einem linearen Polyolefin-Polymer, und weiteren inneren Lagen, hergestellt aus Barrierematerialien gegenüber Feuchtigkeit, Gasen und UV(ultraviolettes)-Licht.

3. Arzneimittel-/Medizinproduktsystem nach Anspruch 1 oder 2, das einen schützenden Aufbewahrungskasten umfasst, der aus Pappe oder geschäumtem Polystyrol oder anderen Schaumplatten hergestellt ist.

4. Arzneimittel-/Medizinproduktsystem nach einem der Ansprüche 1 bis 3, wobei der Aufbewahrungsbeutel innere Streifen umfasst, vorzugsweise innere Mikrostreifen, oder eine beliebige andere Prägung oder Textur, die die Innenfläche in Kontakt mit dem Arzneimittel nicht vollständig flach macht.

5. Arzneimittel-/Medizinproduktsystem nach einem der Ansprüche 1 bis 4, wobei der Aufbewahrungsbeutel für eine tragbare Verwendung angepasst ist, und bevorzugt wobei ein einzelner Aufbewahrungsbeutel mit einer individuellen äußeren Verpackung ausgerüstet ist und/oder mehrere Aufbewahrungsbeutel mit einer gemeinsamen Verpackung ausgerüstet sind.

6. Arzneimittel-/Medizinproduktsystem nach einem der Ansprüche 1 bis 5, wobei der Aufbewahrungsbeutel oder das Aufbewahrungsbeutelset die Form einer Arzneimittelkassette hat.

7. Arzneimittel-/Medizinproduktsystem nach einem der Ansprüche 1 bis 6, wobei das analgetische Arzneimittel ausgewählt ist aus der Gruppe von analgetischen pharmazeutischen Substanzen wie Morphin, Oxycodon, Fentanyl, Sufetanil, Pethidin, und/oder das anästhetische Arzneimittel ausgewählt ist aus der Gruppe der Aminoamidgruppe von anästhetischen pharmazeutischen Substanzen wie Ropivacain, Lidocain, Bupivacain, und/oder aus der esterbasierten Gruppe von anästhetischen pharmazeutischen Substanzen wie etwa Chloroprocain.

8. Arzneimittel-/Medizinproduktsystem nach einem der Ansprüche 1 bis 7, wobei das System eine Patienten-Rückmeldungsfunktion und eine Schnittstelle umfasst, durch die die analgetische Wirkung von dem Patienten als ein Wert innerhalb eines Bereichs, oder als eine Antwort auf komplexere Fragen berichtet wird, die von der Rückmeldungsfunktion gestellt werden.

9. Arzneimittel-/Medizinproduktsystem nach einem der Ansprüche 1 bis 8, wobei das System eine Steuerungsfunktion zur Erkennung des Patienten und zur Überprüfung des Verabreichungswegs vordem Beginn der Verabreichung und zur Bereitstellung eines entsprechenden Kontrollberichts umfasst.

10. Arzneimittel-/Medizinproduktsystem nach einem der Ansprüche 1 bis 9, wobei das System eine Sicherheitsfunktion zur Überwachung des Atemrhythmus mit einer Anzeige und/oder Warnung im Fall der Verabreichung einer falschen Dosis umfasst.

11. Arzneimittel-/Medizinproduktsystem nach Anspruch 1, wobei das System ein Schutzmittel gegen Missbrauch umfasst, und wobei die Erkennung des Dateninhalts des/der Aufbewahrungsbeuteletiketts/-kennzeichnung durchgeführt wird, vorzugsweise automatisch, durch eine Funktion der Pumpe, durch eine mobile Anwendung, die vor Ort mit der Pumpe verbunden ist oder mit dieser kommuniziert, oder durch eine Internetanwendung, die vor Ort mit der Pumpe verbunden ist oder mit dieser kommuniziert, und wobei die integrierte Funktion der Pumpe, der mobilen oder der Internetanwendung befähigt ist, den Aufbewahrungsbeutel, vorzugsweise die Arzneimittelkassette, zu entriegeln, um bereit zur Verwendung zu sein.

12. Arzneimittel-/Medizinproduktsystem nach Anspruch 11, wobei das System eine Schnittstellenfunktion umfasst, mittels derer das Verabreichungsprotokoll unter anderem, ohne darauf beschränkt zu sein, die Patientenerkennung, den Verabreichungsweg, die Arzneimitteletikett-Inhaltserkennung, das zwischenzeitliche und/oder endgültige Infusionsprotokoll, die Überwachung des Analgetikaspiegels und/oder andere Nebenwirkungsinformationen und die therapeutischen Ergebnisse über ein Netzwerk vor Ort oder das Internet an den behandelnden Arzt berichtet werden kann, und mittels derer der behandelnde Arzt innerhalb von vorher festgelegten Grenzen Änderungen des Verabreichungsprotokolls bewirken kann.

## Revendications

1. Système de dispositif médicamenteux/médical pour l'administration d'un médicament analgésique et/ou anesthésique à un patient de préférence via la voie IV ou le bloc nerveux central ou le bloc nerveux périphérique comprenant
(a) une pompe pour l'administration d'au moins un médicament analgésique et/ou anesthésique,
(b) une poche de stockage ou un ensemble poche de stockage prérempli(e) avec l'au moins un médicament analgésique et/ou anesthésique,
et **caractérisé en ce que**
le système de dispositif médicamenteux/médical comprend une poche de stockage de médicament préremplie d'un médicament pharmaceutiquement ou dans une installation de préparation pharmaceutique, et dans lequel la poche de stockage préremplie de médicament a une stabilité à long terme avec une durée de conservation de l'ensemble/de la poche prérempli(e) de médicament d'au moins 1 mois ;
et dans lequel la poche de stockage de médicament est personnalisée pour une pompe ou un groupe de pompes spécifique par au moins un moyen par lequel la pompe (a) et la poche de stockage ou l'ensemble poche de stockage (b) soient ajustés pour coopérer exclusivement l'un avec l'autre lors de l'administration à un patient individuel, **en ce que** le système comprend une étiquette/un marquage, fournissant des informations de manière visuelle et/ou sans fil au système de pompe et aux services de support, directement ou par l'intermédiaire d'Internet, **en ce que** la poche de stockage de (b) comprend une étiquette/un marquage d'identification par code-barres en 1D ou 2D et/ou d'identification sans fil RFID/NFC d'une ou plusieurs données associées (i) au type de médicament et à la concentration de médicament, (ii) au solvant, (iii) au volume de la poche, (iv) au lot de médicament, (v) à la voie d'administration, (vi) aux limites maximales pour la perfusion sûre selon le code patient, et facultativement pour (vii) la transmission sans fil du protocole de perfusion ; et dans lequel la fourniture biunivoque directe d'informations à la pompe depuis l'étiquette/le marquage de médicament, concernant (i) le type et la concentration de médicament permet le choix correct et/ou automatique du protocole de perfusion ;
et dans lequel le terme « prérempli » signifie que la poche ou l'ensemble poche est rempli du médicament analgésique et/ou anesthésique par une compagnie pharmaceutique et qu'elle (il) peut être conservé(e) et/ou fourni(e) par gestion de la chaîne d'approvisionnement pharmaceutique classique, et dans lequel la poche de stockage préremplie ou un ensemble poche de stockage (b) est fourni (e) conformément aux lignes directrices d'une autorité de régulation des médicaments compétente applicables pour les systèmes préremplis pour le stockage à long terme.

2. Système de dispositif médicamenteux/médical selon la revendication 1, dans lequel la poche de stockage est composée d'un film multicouche qui a une surface en contact avec le médicament composée d'un matériau sélectionné dans le groupe de l'éthylènevinylacétate (EVA), et un polymère polyoléfinique linéaire, et/ou un polymère polyoléfinique cyclique, de préférence d'un polymère polyoléfinique linéaire ; et en outre des couches internes composées de matériaux formant barrière à l'humidité, aux gaz et à la lumière UV.

3. Système de dispositif médicamenteux/médical selon la revendication 1 ou 2, comprenant un boîtier de stockage de protection composé de carton ou de polystyrène expansé ou d'autres feuilles de mousse.

4. Système de dispositif médicamenteux/médical selon l'une quelconque des revendications 1 à 3, dans lequel la poche de stockage comprend des stries internes, de préférence des micro-stries internes, ou tout autre relief ou texture qui rend la surface en contact avec le médicament pas complètement plate.

5. Système de dispositif médicamenteux/médical selon l'une quelconque des revendications 1 à 4, dans lequel la poche de stockage est ajustée pour une utilisation portable, et de préférence dans lequel une poche de stockage unique est équipée d'un emballage externe individuel et/ou une multitude de poches de stockages est équipée d'un emballage collectif.

6. Système de dispositif médicamenteux/médical selon l'une quelconque des revendications 1 à 5, dans lequel la poche de stockage ou l'ensemble poche de stockage se trouve sous la forme d'une cassette de médicament.

7. Système de dispositif médicamenteux/médical selon l'une quelconque des revendications 1 à 6, dans lequel le médicament analgésique est sélectionné dans le groupe des substances pharmaceutiques analgésiques telles que la morphine, l'oxycodone, le fentanyl, le sufentanil, la péthidine, et/ou le médicament anesthésique est sélectionné dans le groupe du groupe de substances pharmaceutiques anesthésiques amino-amide telles que la ropivacaïne, la lidocaïne, la bupivacaïne, et/ou dans le groupe de substances pharmaceutiques anesthésiques à base d'ester telles que la chloroprocaïne.

8. Système de dispositif médicamenteux/médical selon l'une quelconque des revendications 1 à 7, dans lequel le système comprend une fonction de rétroaction du patient et une interface par laquelle l'effet analgésique est rapporté par le patient sous la forme d'une valeur dans une plage, ou sous la forme d'une réponse à des questions plus complexes posées par la fonction de rétroaction.

9. Système de dispositif médicamenteux/médical selon l'une quelconque des revendications 1 à 8, dans lequel le système comprend une fonction de contrôle pour l'identification du patient et la vérification de la voie d'administration avant le début de l'administration, et pour fournir un rapport de contrôle correspondant.

10. Système de dispositif médicamenteux/médical selon l'une quelconque des revendications 1 à 9, dans lequel le système comprend une fonction de sécurité pour la surveillance de la fréquence respiratoire avec une indication et/ou un avertissement en cas d'administration d'une dose incorrecte.

11. Système de dispositif médicamenteux/médical selon la revendication 1, dans lequel le système comprend un moyen de protection contre les abus et dans lequel l'identification du contenu de données de l'étiquette/du marquage de poche de stockage est réalisée, de préférence automatiquement, par une fonction de la pompe, par une application mobile raccordée à la pompe ou communiquant avec celle-ci localement, ou par une application Internet raccordée à la pompe ou communiquant avec celle-ci localement, et dans lequel la fonction intégrée de la pompe, l'application mobile ou Internet est activée pour débloquer la poche de stockage, de préférence la cassette de médicament, pour qu'elle soit prête à l'utilisation.

12. Système de dispositif médicamenteux/médical selon la revendication 11, dans lequel le système comprend une fonction d'interface par l'intermédiaire de laquelle le protocole d'administration, comprenant de manière non restrictive, l'identification du patient, la voie d'administration, l'identification du contenu de l'étiquette de médicament, le protocole de perfusion intermédiaire et/ou final, la surveillance du niveau d'analgésie et/ou d'autres informations relatives aux effets secondaires, et les résultats thérapeutiques peuvent être rapportés à un réseau local ou sur Internet pour le médecin traitant, et par l'intermédiaire de laquelle le médecin traitant peut apporter des modifications au protocole d'administration dans des limites prédéfinies.
